# EUROPÄISCHE PATENTSCHRIFT

(11) **EP 1 125 563 B1**
(45) Veröffentlichungstag und Bekanntmachung des Hinweises auf die Patenterteilung: **19.10.2005**
(21) Anmeldenummer: 01101497.4
(22) Anmeldetag: 24.01.2001
(51) Int. Cl.: A61F 2/34, A61L 27/02

(54) **Pressfit Cup mit Dämpfungseigenschaften**
Press fit cup with damping properties
Cupule insérée à force avec propriétés d'amortissement

(30) Priorität: 27.01.2000 DE 10003543
(43) Veröffentlichungstag der Anmeldung: 22.08.2001
(73) Patentinhaber: Draenert, Klaus, Prof.Dr.med., 81545 München (DE)
(72) Erfinder: Draenert, Klaus, Prof.Dr.med., 81545 München (DE)

(56) Entgegenhaltungen:
- EP-A- 0 105 098
- EP-A- 0 265 712
- EP-A- 0 617 932
- EP-A- 0 636 351
- EP-A- 0 829 244
- EP-A- 0 901 777
- EP-A- 0 974 316
- WO-A-94/23670
- WO-A-99/43274
- FR-A- 2 660 546
- FR-A- 2 688 402
- US-A- 4 662 891
- US-A- 4 795 469
- US-A- 5 725 591
- US-A- 5 928 288

## Beschreibung

Beim künstlichen Hüftgelenksersatz wurden am Anfang Versuche mit Implantaten gemacht, die frei im Knochen verankert waren (Smith-Peterson 1948; Gebrüder Judet 1950). Diese Versuche schlugen fehl und wurden durch die alles überragenden Implantationen von John Charnley (1960), bei denen sowohl die Komponente im Oberschenkelknochen, als auch die im Becken, mit einem selbsthärtenden Knochenzement verankert worden war; diese Knochenzemente waren durchwegs kalthärtende Polymethylmetacrylate (PMMA). Die standardisierte Zementauffüllung und vergleichbare postoperative Ergebnisse waren jedoch nicht leicht zu erreichen (Draenert et al. "Manual of Cementing Technique" 1998). Es wurden daher immer wieder Versuche unternommen, Implantate ohne Knochenzement im Knochen zu verankern. Grundlagenforschungen haben bewiesen, daß die Krafteinleitung vom Implantat in den Knochen, aber auch die Deformation des Knochens am Implantat durch die den Knochen umgebende Muskulatur für die knöcherne Integration verantwortlich zu machen waren, in dem nämlich große Deformationen zu Relativbewegungen führen, die den Knochenabbau stimulieren und die Bildung von Bindegewebe induzieren. Wenn diese Deformation allerdings gleichförmig um ein Implantat stattfindet, kann das Design des Implantates durch Vorspannung diese Deformation auffangen (Galante), was im Bereich der Pfanne des Hüftgelenkes auch erfolgreich versucht worden war.

### Stand der Forschung

Es ist Stand der Forschung, daß Femurkomponenten in aller Regel zementiert werden und die Pfannenkomponente häufig zementfrei im sog. pressfit-Verfahren verankert wird. Die Verfahren sind nicht ganz einheitlich. Von Morscher (1983) ist eine HDPE-pressfit Pfanne bekannt, die auf der Rückseite mit Titannetzen beschichtet ist und eine gewisse Elastizität besitzt, die jedoch als artikulierende Gleitpaarung Polyäthylen/Metall oder Polyäthylen/Keramik vorsieht. Diese pressfit-Pfanne hat als hervorstechenden Nachteil das Material Polyäthylen, welches einen wesentlich höheren Abriebkoeffizienten als beispielsweise die Gleitpaarung Keramik/Keramik hat. Von Galante ist eine HDPE/Titan-Pfanne bekannt, welche auch pressfit verankert wird, mit einem Titanfilz beschichtet ist, die jedoch zusätzlich noch exzentrisch mit Schrauben im Beckenknochen verankert wird. Hieraus ergibt sich neben dem Abriebproblem Polyäthylen/Metall noch ein weiteres, nämlich das der exzentrischen Verschraubung, welche gegenüber dem Knochenlager zur Aufhebung der pressfit-Vorspannung führt. Ein Spätproblem dieser pressfit-Pfannen nach Galante war die starke und ungleiche Atrophie im Pfannendach, die diese Pfannen und alle ähnlichen, steifen pressfit-Pfannen begleitete. Alle anderen zementfreien Pfannen sind entweder sog. "Ingrowth"-Oberflächen, oder Schraubpfannen, d. h. Pfannen, die zirkulär ein Gewinde aufweisen, welches in den Beckenknochen hineingeschraubt wird, zu sehr hohen Spannungsspitzen führt und in der Regel von Bindegewebe abgescheidet wird. Da auch von den zementierten Pfannen sehr unterschiedliche Ergebnisse berichtet wurden (Morscher 1983, Schwedenstudie 1996), war ein Bedarf gegeben, die guten Ergebnisse von pressfit-verankerten Pfannen weiter zu verbessern, die Implantations- technik einheitlicher zu gestalten und in der Konstruktion der Pfannen nach Wegen zu suchen, die starke und ungleichmäßige Atrophie zu verhindern. Dieses Ziel konnte mit der nachfolgenden Erfindung in allen Belangen zufriedenstellend geregelt werden. Der der Erfindung nächstliegende Stand der Technik ist in dem Dokument WO-A-99 43 274 beschrieben.
* Morscher E. (1983) Die zementierte Fixation von Hüftendoprothesen. Berlin - Heidelberg - New York - Tokyo: Springer.
   Malchau H., Herberts P. (1996) Prognosis of total hip replacement. Surgical and cementing technique in THR: A tension-risk study of 134.056 primary operations. Presented at 63^{rd} Annual Meeting of the American Academy of Orthopaedic Surgeons 22-26 Februar, Atlanta.

### Beschreibung

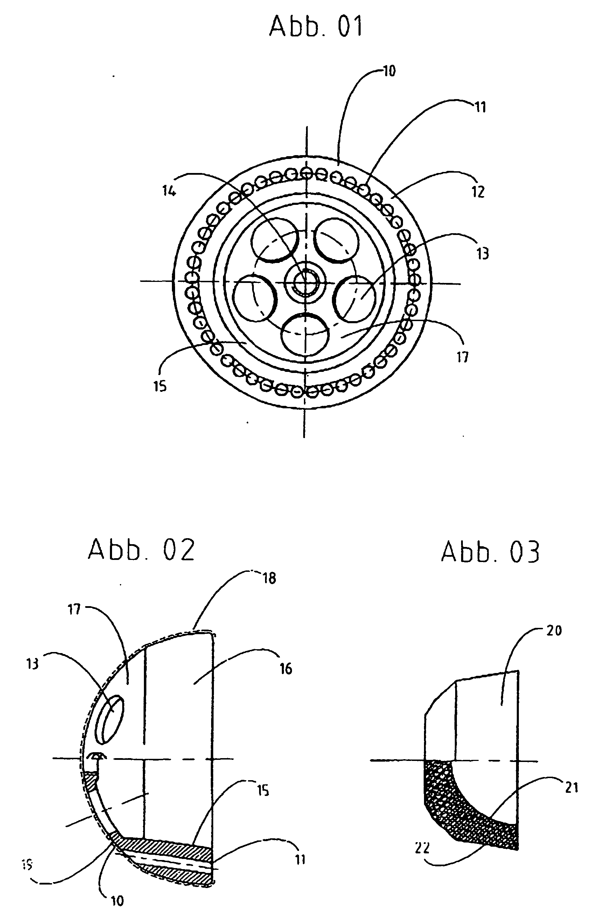

In den Abbildungen 1 bis 3 ist ein zweiteiliges Implantat dargestellt, welches aus einer Titanschale (10) und einem Keramikinsert (20) besteht.

Die Titanschale läßt deutlich zwei Abschnitte erkennen, einen stabilen Ring (12) mit Bohrungen (11), welche gleichmäßig über die Zirkumferenz verteilt sind und parallel zum Innenkonus (15) der Schale geführt sind, und einen Boden oder eine Kappe (Polkappe, 17), welche von größeren Bohrungen gefenstert ist (13) und eine dünne Wand aufweist (19). Im Zentrum der Halbkugelschale ist im Pol eine Bohrung (14) mit einem Gewinde dargestellt. Dieses dient der Verankerung mit dem Applikationsgerät.

In der Abbildung 2 ist diese Titanschale im Schnitt dargestellt. Die Konusoberfläche der Titanschale (15) und die korrespondierende Oberfläche des Keramikinserts (22) sind so aufeinander abgestimmt, daß ein nahezu 100 % Flächenkontakt besteht.

Im Schnitt ist das äquatoriale Bandsegment (18) deutlich vom dünnwandigeren (19) Polsegment (17) zu unterscheiden. Das Polsegment ist deutlich gegenüber dem Radius des äquatorialen Kugelsegmentes abgeflacht. Die Oberfläche der Titanschale ist stirnseitig glatt (Abb. 1) (12), über der konvexen, dem Knochen zugewandten Seite jedoch rauh (18). Die Rauhigkeit, die nicht zu Relativbewegungen führt, liegt zwischen 80 µm und 100 µm.

Der dünnwandige Boden des Implantates erlaubt eine elastische Deformierung im Polbereich, während die gleichmäßigen Bohrungen parallel zur Konusoberfläche die steife Kraftübertragung von der artikulierenden Keramikoberfläche (21) über die steife Keramik (20) dämpft, so daß die Kraft gleichmäßig in den Knochenring eingeleitet wird.

## Patentansprüche

1. Implantat, bestehend aus einer äußeren Metallschale (10) und einem inneren Einsatz (20) für die artikulierende Gelenkfläche, dessen äußere Schale (10) folgende Merkmale aufweist:
• die Metallschale (10) ist halbkugelförmig oder ellipsoid ausgebildet.
• das Polsegment (17) der Schale (10) ist abgeflacht.
• die Wand des Polkompartimentes (17) weist Aussparungen (13) auf und/oder seine Wand ist verdünnt,
**dadurch gekennzeichnet, daß** das abgeflachte Polsegment (17) in ein bandförmiges, äquatoriales Kugelsegment (18) übergeht, dessen Wand Aussparungen (11) aufweist, die einen steifen inneren Ring dämpfend gegenüber einem steifen äußeren Ring absetzen.

2. Implantat nach Anspruch (1) so beschaffen, daß die innere Begrenzung der Metallschale (10) einen stumpfen Kegelkonus darstellt.

3. Implantat nach Ansprüchen (1) und (2) so beschaffen, daß die Aussparungen (13) des Polsegmentes (17) Bohrungen sind mit einem Durchmesser zwischen 2 mm und 12 mm, vorzugsweise 10 mm.

4. Implantat nach den Ansprüchen (1) bis (3) so beschaffen, daß die Aussparungen des Polsegmentes (17) 30 % bis 70 %, vorzugsweise 50 % bis 60 % der Polwand ausmachen.

5. Implantat nach den Ansprüchen (1) bis (4) so beschaffen, daß die Aussparungen des äquatorialen Kugelsegmentes (18) Bohrungen sind mit einem Durchmesser zwischen 2,5 mm und 9 mm und an Zahl insgesamt 12 bis 60, vorzugsweise 40 bis 50 Bohrungen über die Zirkumferenz aufweisen.

6. Implantat nach den Ansprüchen (1) bis (5) so beschaffen, daß die Bohrungen, bzw. Aussparungen (11) parallel zur inneren Konuswand geführt sind.

7. Implantat nach den Ansprüchen (1) bis (6) so beschaffen, daß die äußere Oberfläche eine Rauhigkeit von 60 µm bis 200 µm, vorzugsweise 80 µm bis 100 µm aufweist.

8. Implantat nach den Ansprüchen (1) bis (7) so beschaffen, daß als Metall Titan oder eine Titanlegierung, Tantal oder eine Tantallegierung oder Kobalt-Chrom-Molybdän verwandt wird.

9. Implantat nach den Ansprüchen (1) bis (8) so beschaffen, daß als innerer Einsatz (20) ein Keramikinsert aus Al₂O₃, ZrO₂ oder HDPE oder verwandtes Kunststoffmaterial verwandt wird.

10. Implantat nach den Ansprüchen (1) bis (9) so beschaffen, daß der innere Einsatz (20) ein Probeinsert aus Kunststoff oder einem sterilisierbaren Material ist, welcher leicht entfernt werden kann und so **gekennzeichnet** ist, beispielsweise durch einen integrierten Draht oder ein Band, daß ein versehentliches Implantieren nicht vorkommen kann.

## Claims

1. An implant consisting of an outer metal shell (10) and an inner insert (20) for the articulating articular surface, whose outer shell (10) has the following features:
- the metal shell (10) is constructed as hemispherical or ellipsoidal,
- the pole segment (17) of the shell (10) is flattened,
- the wall of the pole compartment (17) has recesses (13) and/or its wall is thinner,
**characterised in that**
- the flattened pole segment (17) goes over into a band-shaped equatorial spherical segment (18) whose wall has recesses (11) which position a rigid inner ring against a rigid outer ring in a damping fashion.

2. The implant according to claim 1 **characterised in that** the inner boundary of the metal shell (10) is a truncated spherical cone.

3. The implant according to claims 1 and 2 **characterised in that** the recesses (13) of the pole segment (17) are holes having a diameter between 2 mm and 12 mm, preferably 10 mm.

4. The implant according to claims 1 to 3 **characterised in that** the recesses of the pole segment (17) account for 30% to 70%, preferably 50% to 60% of the pole wall.

5. The implant according to claims 1 to 4, **characterised in that** the recesses of the equatorial spherical segment (18) have a total number of 12 to 60, preferably 40 to 50 holes over the circumference, having a diameter between 2.5 mm and 9 mm.

6. The implant according to claims 1 to 5, **characterised in that** the holes or recesses (11) are guided parallel to an inner cone wall.

7. The implant according to claims 1 to 6, **characterised in that** the outer surface has a roughness of 60 µm to 200 µm, preferably 80 µm to 100 µm.

8. The implant according to claims 1 to 7, **characterised in that** titanium or a titanium alloy, tantalum or a tantalum alloy or cobalt-chromium-molybdenum is used as metal.

9. The implant according to claims 1 to 8, **characterised in that** a ceramic insert made of Al₂O₃, ZrO₂ or HDPE or related plastic material is used as an internal insert (20).

10. The implant according to claims 1 to 9, **characterised in that** the internal insert (20) is a trial insert made of plastic or a sterilisable material which can be easily removed and is **characterised**, for example, by an integrated wire or a strip such than an unintentional implantation cannot occur.

## Revendications

1. Implant composé d'une coque métallique extérieure (10) et d'un insert intérieur (20) pour la surface d'articulation, dont la coque extérieure (10) présente les caractéristiques suivantes :
- la coque métallique (10) est conçue de forme semi-sphérique ou ellipsoïdale,
- le segment de pôle (17) de la coque (10) est aplati,
- la paroi du compartiment de pôle (17) présente des évidements (13) et/ou sa paroi est amincie,
**caractérisé en ce que**
- le segment de pôle aplati (17) se prolonge par un segment sphérique équatorial en forme d'attache (18) dont la paroi présente des évidements (11) qui décalent une bague rigide intérieure de manière amortie par rapport à une bague rigide extérieure.

2. Implant selon la revendication 1, **caractérisé en ce que** la limite intérieure de la coque métallique (10) présente un cône tronqué.

3. Implant selon les revendications 1 et 2, **caractérisé en ce que** les évidements (13) du segment de pôle (17) sont des alésages présentant un diamètre compris entre 2 mm et 12 mm, de préférence de 10 mm.

4. Implant selon l'une quelconque des revendications 1 à 3, **caractérisé en ce que** les évidements du segment de pôle (17) représentent 30 % à 70%, de préférence 50 % à 60 % de la paroi du pôle.

5. Implant selon l'une quelconque des revendications 1 à 4, **caractérisé en ce que** les évidements du segment sphérique équatorial (18) sont des alésages présentant un diamètre compris entre 2,5 mm et 9 mm et présentent un nombre de 12 à 60, de préférence de 40 à 50 alésages au total au-dessus de la circonférence.

6. Implant selon l'une quelconque des revendications 1 à 5, **caractérisé en ce que** les alésages ou évidements (11) sont guidés parallèlement à la paroi de cône intérieure.

7. Implant selon l'une quelconque des revendications 1 à 6, **caractérisé en ce que** la surface extérieure présente une rugosité comprise entre 60 µm et 200 µm, de préférence entre 80 µm et 100 µm.

8. Implant selon l'une quelconque des revendications 1 à 7, **caractérisé en ce que** du titane ou un alliage de titane, du tantale ou un alliage de tantale ou du cobalt-chrome-molybdène sont utilisés en tant que métal.

9. Implant selon l'une quelconque des revendications 1 à 8, **caractérisé en ce qu'**un insert en céramique en Al₂O₃, ZrO₂ ou HDPE ou en un matériau synthétique apparenté est utilisé en tant qu'insert intérieur (20).

10. Implant selon l'une quelconque des revendications 1 à 9, **caractérisé en ce que** l'insert intérieur (20) est un insert d'échantillon en matériau synthétique ou en un matériau pouvant être stérilisé qui peut être retiré facilement et est **caractérisé par** exemple par un fil intégré ou une attache de sorte qu'une implantation involontaire ne puisse pas se produire.
